# EUROPEAN PATENT APPLICATION

(11) **EP 3 909 588 A1**
(43) Date of publication of application: **17.11.2021**
(21) Application number: 20737966.0
(22) Date of filing: 09.01.2020
(51) Int. Cl.: A61K 31/713, A61P 35/00, A61P 37/04, A61M 5/303, C12N 15/117

(54) **IMMUNOSTIMULATING COMPOSITION**

(30) Priority: 10.01.2019 JP 2019002518
(71) Applicant: Osaka University, Suita-shi, Osaka 565-0871 (JP); Daicel Corporation, Osaka 530-0011 (JP)
(72) Inventor: NISHIKAWA, Tomoyuki, Suita-shi, Osaka 565-0871 (JP); KANEDA, Yasufumi, Suita-shi, Osaka 565-0871 (JP); YAMASHITA, Kunihiko, Suita-shi, Osaka 565-0871 (JP); SUZUKI, Ayano, Tokyo 108-8230 (JP); TERAI, Kazuhiro, Tokyo 108-8230 (JP)
(74) Representative: Grünecker Patent- und Rechtsanwälte PartG mbB
(86) International application number: PCT/JP2020/000482
(87) International publication number: WO 2020/145350

(57) **Abstract**

An object of the present disclosure is at least to provide a technique for stimulating immunity in mammals, and the object is fulfilled by double-stranded RNA composed of: RNA composed of a base sequence represented by a sequence A below; and RNA composed of a base sequence represented by a sequence B below, each base of an (N)₁₃₋₂₅ portion in the base sequence represented by the sequence A and the base sequence represented by the sequence B being or not being complementary bases, and all of the bases of (N)₅₋₁₀ portions in the base sequence represented by the sequence A and the base sequence represented by the sequence B being complementary bases, and a Tm value of the (N)₅₋₁₀ portion being 20°C or higher: Sequence A: 5'-UUGUCAUAUGGACAAGUCCAAGACU(N)₁₃₋₂₅(N)₅₋₁₀-3' (SEQ ID No: 1), and Sequence B: 5'-(N)₅₋₁₀(N)₁₃₋₂₅AGUCUUGGACUUGUCCAUAUGACAA-3' (SEQ ID No: 2).

SEQUENCE LISTING

<110>  Osaka University
       Daicel Corporation

<120>  A composition for immunostimulation

<130>  P180493

<150>  JP 2019-002518
<151>  2019-01-10

<160>  9

<170>  PatentIn version 3.5

<210>  1
<211>  60
<212>  RNA
<213>  Artificial Sequence

<220>
<223>  RNA 1


<220>
<221>  misc_feature
<222>  (26)..(60)
<223>  n is a, c, g, or u


<220>
<221>  misc_feature
<222>  (44)..(60)
<223>  either be present or absent

<400>  1
uugucauaug gacaagucca agacunnnnn nnnnnnnnnn nnnnnnnnnn nnnnnnnnnn      60


<210>  2
<211>  60
<212>  RNA
<213>  Artificial Sequence

<220>
<223>  RNA 2


<220>
<221>  misc_feature
<222>  (1)..(35)
<223>  n is a, c, g, or u


<220>
<221>  misc_feature
<222>  (1)..(17)
<223>  either be present or absent

<400>  2
nnnnnnnnnn nnnnnnnnnn nnnnnnnnnn nnnnnagucu uggacuuguc cauaugacaa      60

<210>  3
<211>  25
<212>  RNA
<213>  Artificial Sequence

<220>
<223>  RNA 3

<400>  3
uccagguacc gcggagcuuc gaucg                                          25


<210>  4
<211>  25
<212>  RNA
<213>  Artificial Sequence

<220>
<223>  RNA 4

<400>  4
cgaucgaagc uccgcgguac cugga                                          25


<210>  5
<211>  25
<212>  RNA
<213>  Artificial Sequence

<220>
<223>  RNA 5

<400>  5
uacgacugug gauauauaua aauau                                          25


<210>  6
<211>  57
<212>  RNA
<213>  Artificial Sequence

<220>
<223>  RNA 6

<400>  6
uugucauaug gacaagucca agacuuccag guaccgcgga gcuucgaucg uucugca       57


<210>  7
<211>  57
<212>  RNA
<213>  Artificial Sequence

<220>
<223>  RNA 7

<400>  7
ugcagaauac gacuguggau auauauaaau auagucuugg acuuguccau augacaa       57


<210>  8
<211>  57

<212>  RNA
<213>  Artificial Sequence

<220>
<223>  RNA 8

<400>  8
uugucauaug gacaagucca agacuuccag guaccgcgga gcuucgaucg uucugca          57


<210>  9
<211>  57
<212>  RNA
<213>  Artificial Sequence

<220>
<223>  RNA 9

<400>  9
ugcagaacga ucgaagcucc gcgguaccug gaagucuugg acuuguccau augacaa          57

## Description

### TECHNICAL FIELD

The present disclosure relates to a composition for immunostimulation.

### BACKGROUND ART

There is continuing progress in the diagnosis or treatment of cancer, and various pharmaceuticals for cancer treatment have been developed. A nucleic acid pharmaceutical using antisense nucleic acid is one of the various pharmaceuticals for cancer treatment. For example, compositions used when distributing activators for treating malignant tumors have been developed, and examples of such malignant tumors include malignant tumors located in lungs, kidneys, pancreas, liver, bones, skin, and intestines (colon) (Patent Documents 1 to 3).

The present inventors have developed various molecular therapies for treating cancer or tumors.

As one of such molecular therapies, the present inventors have developed a vector which is an HVJ envelope vector (HVJ-E) capable of transducing genes or siRNA on the basis of inactivated Sendai virus (hemagglutinating virus of Japan, HVJ) particles, and have found that HVJ-E itself has an antitumor effect (Non-patent Documents 1 to 4). In particular, Non-patent Document 1 reports that HVJ-E activates natural killer (NK) cells.

In addition, the present inventors have reported that Sendai virus-derived RNA (IVT-B2-RNA) activates antitumor immunity and selectively induces cell death of cancer cells, and that activation of natural killer (NK) cells due to IVT-B2-RNA is associated with the activation of antitumor immunity and induction of cell death (Non-patent Document 5).

In this manner, nucleic acid pharmaceuticals useful for treatment of cancer or tumors have been developed.

### PRIOR ART DOCUMENTS

### PATENT DOCUMENTS

Patent Document 1: JP 2018-512060 A
Patent Document 2: JP 2018-143248 A
Patent Document 3: JPWO 2017/033912 A1

### NON-PATENT DOCUMENTS

Non-patent Document 1: Cancer Res., 67:227-236 (2007)
Non-patent Document 2: Cancer Immunol. Immunother., 57:73-84 (2008)
Non-patent Document 3: Int. J. Cancer, 124: 2478-2487 (2009)
Non-patent Document 4: Int. J. Cancer, 126:1982-1991 (2010)
Non-patent Document 5: Mol. Therapy, 24(1):135-45 (2016)

### SUMMARY OF THE INVENTION

### PROBLEMS TO BE SOLVED BY THE INVENTION

An object of the present disclosure is at least to provide technology for stimulating immunity in mammals.

### MEANS FOR SOLVING THE PROBLEMS

<1> Double-stranded RNA composed of:
   RNA composed of a base sequence represented by a sequence A below; and
   RNA composed of a base sequence represented by a sequence B below,
   each base of an (N)₁₃₋₂₅ portion in the base sequence represented by the sequence A and the base sequence represented by the sequence B being or not being complementary bases, and
   all of the bases of (N)₅₋₁₀ portions in the base sequence represented by the sequence A and the base sequence represented by the sequence B being complementary bases, and a Tm value of the (N)₅₋₁₀ portion being 20°C or higher:
      Sequence A: 5'-UUGUCAUAUGGACAAGUCCAAGACU(N)₁₃₋₂₅(N)₅₋₁₀-3' (SEQ ID No: 1), and
      Sequence B: 5'-(N)₅₋₁₀(N)₁₃₋₂₅AGUCUUGGACUUGUCCAUAUGACAA-3' (SEQ ID No: 2).
<2> The double-stranded RNA according to <1>, wherein none of the bases of the (N)₁₃₋₂₅ portions are complementary bases.
<3> The double-stranded RNA according to <1>, wherein all of the bases of the (N)₁₃₋₂₅ portions are complementary bases.
<4> The double-stranded RNA according to any one of <1> to <3>, wherein the Tm value is 20°C.
<5> The double-stranded RNA according to <1>, wherein the (N)₁₃₋₂₅ is (N)₂₅.
<6> The double-stranded RNA according to <5>, wherein none of the bases of the (N)₂₅ portions are complementary bases.
<7> The double-stranded RNA according to <5>, wherein all of the bases of the (N)₂₅ portions are complementary bases.
<8> The double-stranded RNA according to any one of <5> to <7>, wherein the (N)₅₋₁₀ is (N)₇.
<9> The double-stranded RNA according to <8>, wherein the Tm value is 20°C.
<10> A pharmaceutical for immunostimulation comprising:
   the double-stranded RNA according to any one of <1> to <9>.
<11> The pharmaceutical according to <10>, wherein the immunostimulation is activation of natural killer cells.
<12> A pharmaceutical for suppressing growth of cancer cells or tumor cells, the pharmaceutical comprising:
   the double-stranded RNA according to any one of <1> to <9>.
<13> Double-stranded RNA composed of, or a pharmacologically acceptable salt thereof:
   RNA composed of a base sequence represented by a sequence A below; and
   RNA composed of a base sequence represented by a sequence B,
   the double-stranded RNA or the pharmacologically acceptable salt thereof having an immunostimulatory activity or an activity of suppressing growth of cancer cells or tumor cells,
   each base of an (N)₁₃₋₂₅ portion in the base sequence represented by the sequence A and the base sequence represented by the sequence B being or not being complementary bases, and
   all of the bases of (N)₅₋₁₀ portions in the base sequence represented by the sequence A and the base sequence represented by the sequence B being complementary bases, and a Tm value of the (N)₅₋₁₀ portion being 20°C or higher:
      Sequence A: 5'-UUGUCAUAUGGACAAGUCCAAGACU(N)₁₃₋₂₅(N)₅₋₁₀-3' (SEQ ID No: 1), and
      Sequence B: 5'-(N)₅₋₁₀(N)₁₃₋₂₅AGUCUUGGACUUGUCCAUAUGACAA-3' (SEQ ID No: 2).
<14> An injector injecting a solution containing biomolecules into an injection target from an injector body without performing injection through a predetermined structure in a state where the predetermined structure is inserted into the injection target, the injector comprising:
   an accommodation unit that accommodates a solution containing biomolecules; and
   a nozzle portion having an ejection port, through which a pressurized solution containing the biomolecules flows and is ejected to the injection target, wherein
   the solution containing the biomolecules is a solution containing the double-stranded RNA according to any one of <1> to <9>, the pharmaceutical according to any one of <10> to <12>, or a solution containing the double-stranded RNA or a pharmacologically acceptable salt thereof according to <13>.

### EFFECT OF THE INVENTION

The present disclosure may at least have the effect of providing technology for stimulating immunity in mammals.

### BRIEF DESCRIPTION OF THE DRAWINGS

Fig. 1 is a diagram showing a schematic configuration of an injector according to an embodiment.
Fig. 2 is a graph showing results of tumor cell growth suppression test according to an embodiment.

### MODE FOR CARRYING OUT THE INVENTION

An embodiment is a double-stranded RNA composed of: RNA composed of a base sequence represented by a sequence A below; and RNA composed of a base sequence represented by a sequence B below. However, the upper limit of the following (N)₁₃₋₂₅ portion may exceed 25 as long as the double-stranded RNA has an activity of stimulating immunity in mammals to which the double-stranded RNA is administered. The upper limit thereof may be 13 or more.

Sequence A: 5'-UUGUCAUAUGGACAAGUCCAAGACU(N)₁₃₋₂₅(N)₅₋₁₀-3' (SEQ ID No: 1)

Sequence B: 5'-(N)₅₋₁₀(N)₁₃₋₂₅AGUCUUGGACUUGUCCAUAUGACAA-3' (SEQ ID No: 2)

Each base of the (N)₁₃₋₂₅ portion in the base sequence represented by the SEQ ID No: 1 and the base sequence represented by the SEQ ID No: 2 may or may not be a complementary base as long as the double-stranded RNA has an activity of stimulating immunity in mammals to which the double-stranded RNA is administered.

(N)₁₃₋₂₅ in the base sequence represented by the SEQ ID No: 1 is not particularly limited as long as double-stranded RNA composed of the base sequence represented by the SEQ ID No: 1 and the base sequence represented by the SEQ ID No: 2 has an activity of stimulating immunity of mammals to which the double-stranded RNA is administered, but is (N)₂₅ in one preferred embodiment.

In addition, (N)₁₃₋₂₅ in the base sequence represented by the SEQ ID No: 2 is not particularly limited as long as double-stranded RNA composed of the base sequence represented by the SEQ ID No: 1 and the base sequence represented by the SEQ ID No: 2 has an activity of stimulating immunity of mammals to which the double-stranded RNA is administered, but is (N)₂₅ in one preferred embodiment.

The base sequence of (N)₁₃₋₂₅ in the base sequence represented by the SEQ ID No: 1 is not particularly limited as long as double-stranded RNA composed of the base sequence represented by the SEQ ID No: 1 and the base sequence represented by the SEQ ID No: 2 has an activity of stimulating immunity of mammals to which the double-stranded RNA is administered, but is a base sequence represented by the following sequence C in one preferred embodiment.

Sequence C: 5'- UCCAGGUACCGCGGAGCUUCGAUCG -3' (SEQ ID No: 3)

At this time, in a case where all of bases of the (N)₁₃₋₂₅ portions in the base sequence represented by the SEQ ID No: 1 and the base sequence represented by the SEQ ID No: 2 are complementary bases, the base sequence of the (N)₁₃₋₂₅ portion of the base sequence represented by the SEQ ID No: 2 is a base sequence represented by the following sequence D.

Sequence D: 5'-CGAUCGAAGCUCCGCGGUACCUGGA-3' (SEQ ID No: 4)

In addition, in a case where all of bases of the (N)₁₃₋₂₅ portions in the base sequence represented by the SEQ ID No: 1 and the base sequence represented by the SEQ ID No: 2 are not complementary bases, although the base sequence of the (N)₁₃₋₂₅ portion in the base sequence represented by the SEQ ID No: 2 is not particularly limited, examples thereof include a base sequence represented by the following sequence E as long as double-stranded RNA composed of the base sequence represented by the SEQ ID No: 1 and the base sequence represented by the SEQ ID No: 2 has an activity of stimulating immunity in mammals to which the double-stranded RNA is administered.

Sequence E: 5'-UACGACUGUGGAUAUAUAUAAAUAU-3' (SEQ ID No: 5)

The "immunostimulation" in one embodiment means that greater immunity is stimulated in a case where the double-stranded RNA is administered to mammals than in a case where the double-stranded RNA is not administered thereto. In the present specification, such double-stranded RNA is described as "double-stranded RNA having an activity of stimulating immunity in mammals", "double-stranded RNA having an immunostimulatory activity", or the like.

The "immunostimulation" in one embodiment means that natural killer (NK) cells in one preferred embodiment are activated to a greater degree in a case where the double-stranded RNA is administered to mammals than in a case where the double-stranded RNA is not administered thereto.

The activation of natural killer (NK) cells can be confirmed according to a usual method. For example, the activation thereof can be confirmed through an immunostaining method in which a natural killer (NK) cell activation marker is used as an antigen, or the like. Specific examples thereof include flow cytometry. In addition, the activation thereof can be confirmed by performing natural killer (NK) cell cytotoxicity analysis in which target cancer cells and natural killer (NK) cells are co-cultured to measure an ability of natural killer (NK) cells to damage the cancer cells. The immunostaining method can also be performed in, for example, pathological analysis after administration of the double-stranded RNA. For example, pathological analysis can be performed on excised tissue using the immunostaining method.

In addition, the "immunostimulation" in one embodiment means that growth of cancer cells or tumor cells in one preferred embodiment is suppressed to a greater extent in a case where the double-stranded RNA is administered to mammals having cancer or tumors than in a case where the double-stranded RNA is not administered thereto. Suppression of the growth of cancer cells or tumor cells means that the rate of increase in tumor diameter is reduced and means that the rate in one preferred embodiment is zero or close to zero. The diameter of the tumors can be measured through a usual method.

Examples of cancer cells or tumor cells in one embodiment include cancer cells or tumor cells in skin cancer (for example, malignant melanoma), colorectal cancer, lung cancer, ovarian cancer, prostate cancer, breast cancer, renal cancer, liver cancer, pancreatic cancer, gastric cancer, uterine cancer, laryngeal cancer, pharyngeal cancer, tongue cancer, glioma, retinoblastoma, lymphoma, and the like.

Examples of "mammals" in one embodiment include humans, mice, rats, guinea pigs, hamsters (for example, Chinese hamsters), and monkeys (for example, African green monkeys).

In addition, all of the bases of the (N)₅₋₁₀ portions in the base sequence represented by the SEQ ID No: 1 and the base sequence represented by the SEQ ID No: 2 are complementary bases.

(N)₅₋₁₀ in the base sequence represented by the SEQ ID No: 1 is not particularly limited as long as double-stranded RNA composed of the base sequence represented by the SEQ ID No: 1 and the base sequence represented by the SEQ ID No: 2 has an activity of stimulating immunity in mammals to which the double-stranded RNA is administered, but is (N)₇ in one preferred embodiment.

In addition, (N)₅₋₁₀ in the base sequence represented by the SEQ ID No: 2 is not particularly limited as long as double-stranded RNA composed of the base sequence represented by the SEQ ID No: 1 and the base sequence represented by the SEQ ID No: 2 has an activity of stimulating immunity in mammals to which the double-stranded RNA is administered, but is (N)₇ in one preferred embodiment.

The base sequence of the (N)₅₋₁₀ portion in the base sequence represented by the SEQ ID No: 1 in one preferred embodiment is a base sequence represented by the following sequence.

Sequence: 5'-UUCUGCA-3'

At this time, the base sequence of the (N)₅₋₁₀ portion in the base sequence represented by the SEQ ID No: 2 is a base sequence represented by the following sequence.

Sequence: 5'-UGCAGAA-3'

In addition, a Tm value of the (N)₅₋₁₀ portion is not particularly limited as long as the double-stranded RNA has an activity of stimulating immunity in mammals to which the double-stranded RNA is administered, but is usually 20°C or higher and 20°C in one preferred embodiment. On the other hand, the upper limit thereof is not particularly limited as long as the double-stranded RNA has an activity of stimulating immunity in mammals to which the double-stranded RNA is administered, but is, for example, 50°C or lower. In the present disclosure, the Tm value (°C) is the sum of melting temperatures of base pairs of guanine (G) and cytosine (C) and base pairs of adenine (A) and uracil (U) when setting 4°C per base pair of guanine (G) and cytosine (C) and 2°C per base pair of adenine (A) and uracil (U).

RNA composed of the base sequence represented by the SEQ ID No: 1 and RNA composed of the base sequence represented by the SEQ ID No: 2 can be produced through a well-known genetic engineering technique or molecular biological technique. In addition, RNA composed of the base sequence represented by the SEQ ID No: 1 and RNA composed of the base sequence represented by the SEQ ID No: 2 can be produced through nucleic acid synthesis.

An another embodiment is a pharmaceutical for immunostimulation comprising the double-stranded RNA.

Since natural killer (NK) cells are activated to a greater extent in a case where the double-stranded RNA is administered to mammals than in a case where the double-stranded RNA is not administered thereto as described above, the pharmaceutical of this aspect can be used for treating diseases that can be treated by the activation of natural killer (NK) cells. Examples of the treatment include treatment of cancer or tumors.

In addition, since growth of cancer cells or tumor cells is suppressed to a greater degree in a case where the double-stranded RNA is administered to mammals having cancer or tumors than in a case where the double-stranded RNA is not administered thereto as described above, the pharmaceutical of this aspect can be used for treating diseases that can be treated through suppressing the growth of cancer cells or tumor cells. Examples of the treatment include treatment of cancer or tumors.

Examples of cancer or tumors include skin cancer (for example, malignant melanoma), colorectal cancer, lung cancer, ovarian cancer, prostate cancer, breast cancer, renal cancer, liver cancer, pancreatic cancer, gastric cancer, uterine cancer, laryngeal cancer, pharyngeal cancer, tongue cancer, glioma, retinoblastoma, and lymphoma.

The pharmaceutical of this aspect may be formulated using the double-stranded RNA alone as an active component, or may be formulated through a well-known formulation method in which a pharmacologically acceptable carrier or the like is incorporated in addition to the double-stranded RNA.

Examples of formulation materials include a surfactant, an excipient, a colorant, a flavoring agent, a preservative, a stabilizer, a buffer agent, a suspending agent, an isotonic agent, a binder, a disintegrator, a lubricant, a fluidity promoter, and a taste masking agent.

In addition, well-known carriers can be used. Specific examples thereof include light anhydrous silicic acid, lactose, crystalline cellulose, mannitol, starch, carmellose calcium, carmellose sodium, hydroxypropyl cellulose, hydroxypropyl methylcellulose, polyvinyl acetal diethylaminoacetate, polyvinyl pyrrolidone, gelatin, medium-chain fatty acid triglycerides, sucrose, carboxymethyl cellulose, corn starch, and inorganic salts.

The pharmaceutical of this aspect may be powder or a liquid, and other appropriate dosage forms can be selected. In a case where the pharmaceutical is a liquid, the content of the double-stranded RNA in the total amount of the pharmaceutical of this aspect is not particularly limited as long as the double-stranded RNA has an activity of stimulating immunity in mammals to which the double-stranded RNA is administered, but the total amount of the double-stranded RNA is, in view of circumstances such as dissolution or preservation, 1 µg or more in one preferred embodiment, 2 µg or more in another preferred embodiment, and 3 µg or more in still another preferred embodiment, and on the other hand, 6 µg or less in one preferred embodiment, 5 µg or less in another preferred embodiment, and 4 µg or less in still another preferred embodiment. The concentration of the pharmaceutical in the case where the pharmaceutical is used as a liquid may be appropriately adjusted.

A method for applying the pharmaceutical of this aspect to mammals may be oral administration or parenteral administration, but is parenteral administration in one preferred embodiment and injection administration in another preferred embodiment. Examples of injection administration include intraperitoneal injection, intravenous injection, intramuscular injection, and subcutaneous injection, and injection administration can be performed systemically or locally thereby. In addition, the administration method can be appropriately selected depending on the age and symptoms of a patient.

The dosage can be appropriately selected depending on the age, weight, and symptoms of a patient, an administration route, an administration schedule, a dosage form, the strength of an immunostimulatory activity, and the like. For example, the daily dose may be administered once a day or may be administered in several sub-divided doses.

An embodiment is double-stranded RNA composed of, or a pharmacologically acceptable salt thereof:
RNA composed of a base sequence represented by a sequence A below; and
RNA composed of a base sequence represented by a sequence B,
the double-stranded RNA or the pharmacologically acceptable salt thereof having an immunostimulatory activity or an activity of suppressing growth of cancer cells or tumor cells,
each base of an (N)₁₃₋₂₅ portion in the base sequence represented by the sequence A and the base sequence represented by the sequence B being or not being complementary bases, and
all of the bases of (N)₅₋₁₀ portions in the base sequence represented by the sequence A and the base sequence represented by the sequence B being complementary bases, and a Tm value of the (N)₅₋₁₀ portion being 20°C or higher:
   Sequence A: 5'-UUGUCAUAUGGACAAGUCCAAGACU(N)₁₃₋₂₅(N)₅₋₁₀-3' (SEQ ID No: 1), and
   Sequence B: 5'-(N)₅₋₁₀(N)₁₃₋₂₅AGUCUUGGACUUGUCCAUAUGACAA-3' (SEQ ID No: 2).

For details, the description of the aspect is cited.

A salt of a pharmacologically acceptable acid (for example, an inorganic acid or an organic acid) or a base (for example, an alkali metal salt) is used as a pharmacologically acceptable salt, and a pharmacologically acceptable acid addition salt is used as a pharmacologically acceptable salt in one preferred embodiment. Examples of such a salt include a salt of an organic acid (for example, hydrochloric acid, phosphoric acid, hydrobromic acid, or sulfuric acid) or a salt of an organic acid (for example, acetic acid, formic acid, propionic acid, fumaric acid, maleic acid, succinic acid, tartaric acid, citric acid, malic acid, oxalic acid, benzoic acid, methanesulfonic acid, and benzenesulfonic acid). These pharmacologically acceptable salts can be produced through well-known methods.

In addition, the present disclosure includes the following another embodiment.
<1> Use of double-stranded RNA in the manufacture of a pharmaceutical for immuno stimulation.
<2> Use of double-stranded RNA for immunostimulation.
<3> Double-stranded RNA for use in immunostimulation.
<4> Double-stranded RNA for use in the treatment of a disease which can be treated by immunostimulation.
<5> A method for stimulating immunity in a mammal, including a step of administering double-stranded RNA or a pharmaceutical for immunostimulation to a mammal.
<6> A method for treating a disease which can be treated by immunostimulation, including a step of administering double-stranded RNA or a pharmaceutical for immunostimulation to a mammal.
<7> Use of double-stranded RNA in the manufacture of a pharmaceutical for suppressing growth of cancer cells or tumor cells.
<8> Use of double-stranded RNA for suppressing growth of cancer cells or tumor cells.
<9> Double-stranded RNA for use in suppressing growth of cancer cells or tumor cells.
<10> Double-stranded RNA for use in the treatment of a disease which can be treated by suppressing growth of cancer cells or tumor cells.
<11> A method for suppressing growth of cancer cells or tumor cells in a mammal, including a step of administering double-stranded RNA or a pharmaceutical for suppressing growth of cancer cells or tumor cells to a mammal.
<12> A method for treating a disease which can be treated by suppressing growth of cancer cells or tumor cells, including a step of administering double-stranded RNA or a pharmaceutical for suppressing growth of cancer cells or tumor cells to a mammal.

An another embodiment is an injector injecting a solution containing biomolecules into an injection target from an injector body without performing injection through a predetermined structure in a state where the predetermined structure is inserted into the injection target, the injector comprising:
an accommodation unit that accommodates a solution containing biomolecules; and
a nozzle portion having an ejection port, through which a pressurized solution containing the biomolecules flows and is ejected to the injection target, wherein
the solution containing the biomolecules is a solution containing the double-stranded RNA, the pharmaceutical, or a solution containing the double-stranded RNA or the pharmacologically acceptable salt thereof.

The injector according to this aspect injects a solution containing biomolecules to the injection target from an injector main body without performing injection through a given structure in the state where the given structure is inserted into the injection target. The injector may have, for example, a given structure such as a catheter for guiding a solution containing biomolecules from an injector main body to an injection target, for example, when a distance from the injector main body to the injection target is large. Therefore, the injector may or may not have such a given structure. However, when the injector has such a given structure, a solution containing biomolecules is not injected into the injection target in the state where the given structure is inserted into the injection target.

In the injector according to this aspect, a driving unit for pressurizing a solution containing biomolecules is not particularly limited. The pressurization may be caused by, for example, a pressure generated when the pressure of the compressed gas is released, or a pressure generated by combustion of an explosive that is ignited by an ignition device. In addition, the pressure may be a pressure using electrical energy such as from a piezoelectric element or mechanical energy such as from a spring as ejection energy or may be a pressure using an ejection energy generated by appropriately combining these forms of energies. One preferred embodiment is at least an embodiment in which a pressure generated by combustion of explosives ignited by an ignition device is used, and either of the other two pressurization embodiments described above may be used, or both of the other two pressurization embodiments described above may be used in combination therewith.

When a form in which a pressure generated by combustion of an explosive that is ignited by an ignition device is used for pressurization is used, the explosive may be, for example, any explosive among an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO) or an explosive composed of a plurality of combinations of these. Regarding a feature of these explosives, if the combustion products are gases in a high temperature state, since they do not contain gas components at room temperature, the combustion products after ignition immediately condense.

In addition, when the generated energy of a gas generating agent is used as injection energy, various gas generating agents used in a single base smokeless explosive, a gas generator for an airbag, and a gas generator for a seat belt pretensioner can be used as the gas generating agent.

In the injector according to this aspect, the solution containing biomolecules is not accommodated in an accommodation unit from the beginning, and the solution containing biomolecules is accommodated in the accommodation unit by sucking through a nozzle having an injection port. In this manner, when a configuration in which a filling operation in the accommodation unit is required is used, it is possible to inject any required solution containing biomolecules into the injection target. Therefore, in the injector, a syringe part is removable.

Hereinafter, regarding an example of an injector according to this aspect, a syringe 1 (needleless syringe) will be described with reference to the drawings. Note that each of the configurations, combinations thereof, and the like in the embodiments are an example, and various additions, omissions, substitutions, and other changes may be made as appropriate without departing from the spirit of the present invention. The present invention is not limited by the embodiments and is limited only by the claims. The same applies to examples to be described below. Here, the terms "distal end side" and "proximal end side" are used as terms that represent the relative positional relationships in the syringe 1 in the longitudinal direction. The "distal end side" represents a position near the tip of the syringe 1 to be described below, that is, near an injection port 31a, and the "proximal end side" represents a side on the side opposite to the "distal end side" of the syringe 1 in the longitudinal direction, that is, a side on the side of a driving unit 7. In addition, this example is an example in which combustion energy of an explosive that is ignited by an ignition device is used as injection energy and a pharmaceutical (injection) is used as a solution containing biomolecules, but this aspect is not limited thereto.

### (Configuration of syringe 1)

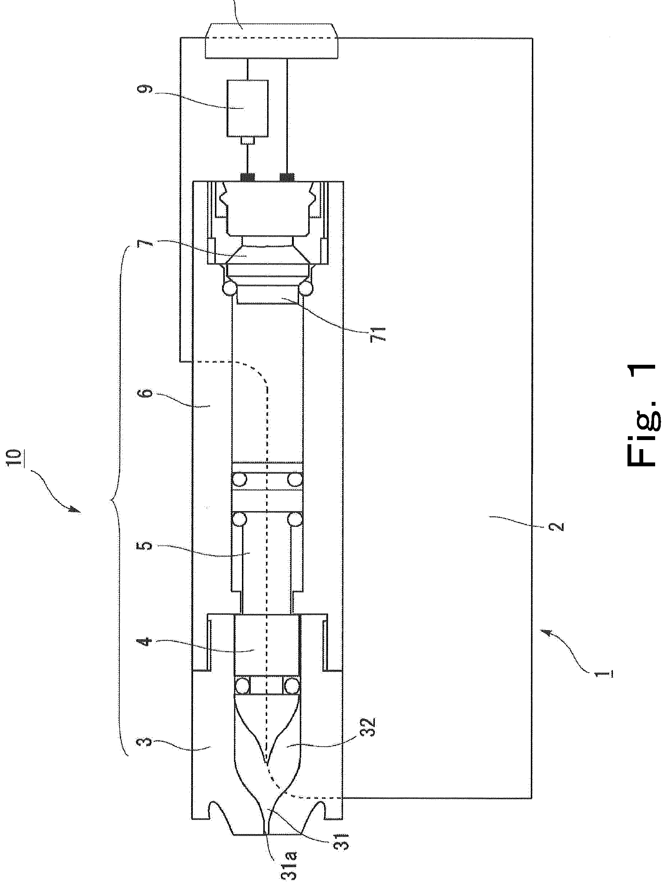

Fig. 1 is a diagram showing a schematic configuration of the syringe 1 and is a cross-sectional view of the syringe 1 in the longitudinal direction. The syringe 1 has a configuration in which a syringe assembly 10 in which a sub-assembly including a syringe part 3 and a plunger 4 and a sub-assembly including a syringe main body 6, a piston 5, and the driving unit 7 are integrally assembled is mounted in a housing (syringe housing) 2.

As described above, the syringe assembly 10 is configured to be detachable from the housing 2. An accommodation unit 32 formed between the syringe part 3 and the plunger 4 included in the syringe assembly 10 is filled with a pharmaceutical (injection), and the syringe assembly 10 is a unit that is discarded whenever the pharmaceutical (injection) is injected. On the other hand, on the side of the housing 2, a battery 9 that supplies power to an igniter 71 included in the driving unit 7 of the syringe assembly 10 is included. When a user performs an operation of pressing a button 8 provided in the housing 2, supply of power from the battery 9 is performed between an electrode on the side of the housing 2 and an electrode on the side of the driving unit 7 of the syringe assembly 10 via a wiring. Here, the shape and position of both electrodes are designed so that the electrode on the side of the housing 2 and the electrode on the side of the driving unit 7 of the syringe assembly 10 are automatically brought in contact when the syringe assembly 10 is mounted in the housing 2. In addition, the housing 2 is a unit that can be repeatedly used as long as power that can be supplied to the driving unit 7 remains in the battery 9. Here, in the housing 2, when the battery 9 has no power, only the battery 9 may be replaced, and the housing 2 may be continuously used.

In addition, in the syringe main body 6 shown in Fig. 1, no particular additional explosive component is provided, but in order to adjust transition of the pressure applied to the pharmaceutical (injection) via the piston 5, a gas generating agent that generates a gas and the like by combustion of a combustion product generated by explosive combustion in the igniter 71 can be provided in the igniter 71 or in a through-hole of the syringe main body 6. A configuration in which a gas generating agent is provided in the igniter 71 is an already known technique as disclosed in WO 01-031282, JP 2003-025950 A, and the like. In addition, regarding an example of a gas generating agent, a single base smokeless explosive including 98 mass% of nitrocellulose, 0.8 mass% of diphenylamine, and 1.2 mass% of potassium sulfate may be exemplified. In addition, various gas generating agents used in a gas generator for an airbag and a gas generator for a seat belt pretensioner can be used. When the dimensions, the size, the shape, and particularly, the surface shape of the gas generating agent when provided in the through-hole is adjusted, it is possible to change a combustion completion time of the gas generating agent, and thus the transition of the pressure applied to the pharmaceutical (injection) can be a desired transition, that is, a transition in which the pharmaceutical (injection) can be appropriately injected into the injection target. In this aspect, the driving unit 7 includes a gas generating agent and the like used as necessary.

### EXAMPLES

Examples will be described below, but none of the examples should be interpreted with a limited meaning.

### [Example 1]

### (Preparation of RNA)

Synthesis of RNA composed of a base sequence represented by a sequence F below and RNA composed of a base sequence represented by a sequence G below was obtained by entrusting this to Gene Design Inc.

The base sequence represented by the following sequence F and the base sequence represented by the following sequence G are sequences in which none of the bases of (N)₁₃₋₂₅ portions is a complementary base and are sequences in which a Tm value of a (N)₅₋₁₀ portion is 20°C.

Sequence F: 5'-UUGUCAUAUGGACAAGUCCAAGACUUCCAGGUA CCGCGGAGCUUCGAUCGUUCUGCA-3' (SEQ ID No: 6)

Sequence G: 5'-UGCAGAAUACGACUGUGGAUAUAUAUAAAUAU AGUCUUGGACUUGUCCAUAUGACAA-3' (SEQ ID No: 7)

### (Annealing of RNA)

Double-stranded RNA composed of RNA composed of the base sequence represented by SEQ ID No: 6 and RNA composed of the base sequence represented by SEQ ID No: 7 was prepared. Specifically, RNA composed of the base sequence represented by SEQ ID No: 6 or RNA composed of the base sequence represented by SEQ ID No: 7 was dissolved in PBS (final concentration of 0.1 µg/µl). An equal amount of the other RNA was mixed therewith, and the mixture was heated at 95°C for 5 minutes using a heat block and then allowed to stand as it was to wait for the temperature to drop to room temperature. Thereafter, the mixture was stored at -20°C until use.

### (Culture of Mouse Malignant Melanoma B16F10 Strain)

A mouse malignant melanoma B16F10 strain (hereinafter, sometimes referred to as a "B16F10 cell") was acquired from American Type Culture Collection (ATCC (address: 12301 Parklawn Drive, Rockville, Maryland 20852, United States of America)). B16F10 cells were cultured in a 5% CO₂ incubator at 37°C using a 10% FBS-containing DMEM medium (Code #. 08458-16, NACALAI TESQUE, INC.) according to a conventional method.

### (Tumor Cell Growth Suppression Test)

1.0 × 10⁶ cells/50 µl of mouse malignant melanoma (B16F10 cells) was seeded in the back skin of a C57BL/6NJcl mouse (female, 7 weeks old), and administration of double-stranded RNA was started when the major axis of an intradermal tumor reached 5 mm. An injector was filled with 30 µl (0.1 µg/µl) of the double-stranded RNA to directly administer the double-stranded RNA to the tumor derived from the B16F10 cells seeded in the back skin. The injector was an injector as shown in Fig. 1 which was set with the condition of 30 mg of an ignition agent ZPP. The double-stranded RNA was administered three times on days 0, 2, and 4, and the diameter of the tumor was measured on the day of the administration and on every other day after the administration until 14 days to calculate the volume of the tumor.

### [Example 2]

The same procedure was followed in the same manner as in Example 1 except that double-stranded RNA composed of RNA composed of a base sequence represented by a sequence H below and RNA composed of a base sequence represented by a sequence I below was used as double-stranded RNA.

The base sequence represented by the following sequence H and the base sequence represented by the following sequence I are sequences in which all of the bases of (N)₁₃₋₂₅ portions are complementary bases and are sequences in which a Tm value of a (N)₅₋₁₀ portion is 20°C.

Sequence H: 5'-UUGUCAUAUGGACAAGUCCAAGACUUCCAGGU ACCGCGGAGCUUCGAUCGUUCUGCA-3' (SEQ ID No: 8)

Sequence I: 5'-UGCAGAACGAUCGAAGCUCCGCGGUACCUGGAA GUCUUGGACUUGUCCAUAUGACAA-3' (SEQ ID No: 9)

### [Reference Example 1]

Frozen HVJ (10,000 HAU) was dissolved and was then inactivated through UV irradiation at 99 mJ/cm². After centrifuging this inactivated HVJ-E solution (20,400 g, 10 min, 4°C), a supernatant was removed, and pellets (HVJ-E) were suspended in PBS (150 µl) to prepare an HVJ-E solution (2,000 HAU/30 µl). The same procedure was followed in the same manner as in Example 1 except that a thus prepared HVJ-E solution was used.

### [Comparative Example 1]

The same procedure was followed in the same manner as in Example 1 except that PBS was used instead of double-stranded RNA.

### [Results]

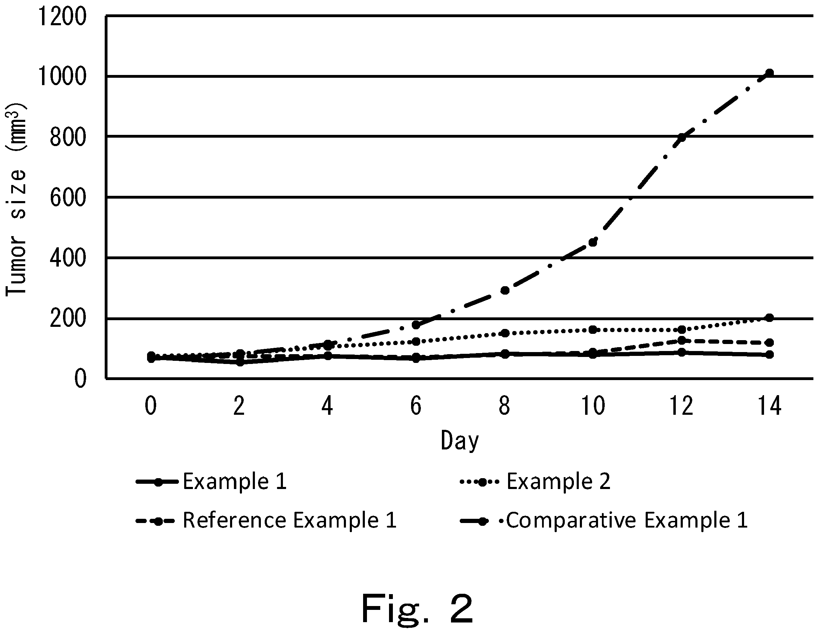

A graph showing diameters of tumors is shown in Fig. 2. The diameter of a tumor increased with the number of days in Comparative Example 1. However, an increase in tumor diameter in Examples 1 and 2 was suppressed in the same manner as in Reference Example 1, and all the cases showed a significant difference (p < 0.05) with Comparative Example 1.

### DESCRIPTION OF REFERENCE NUMERALS

1: Syringe, 2: Housing, 3: Syringe part, 4: Plunger, 5: Piston, 6: Syringe main body, 7: Driving unit, 8: Button, 9: Battery, 10: Syringe assembly, 31: Nozzle, 31a: Injection port, 32: Accommodation unit, 71: Igniter

## Claims

1. Double-stranded RNA composed of:
RNA composed of a base sequence represented by a sequence A below; and
RNA composed of a base sequence represented by a sequence B below,
each base of an (N)₁₃₋₂₅ portion in the base sequence represented by the sequence A and the base sequence represented by the sequence B being or not being complementary bases, and
all of the bases of (N)₅₋₁₀ portions in the base sequence represented by the sequence A and the base sequence represented by the sequence B being complementary bases, and a Tm value of the (N)₅₋₁₀ portion being 20°C or higher:
Sequence A: 5'-UUGUCAUAUGGACAAGUCCAAGACU(N)₁₃₋₂₅(N)₅₋₁₀-3' (SEQ ID No: 1), and
Sequence B: 5'-(N)₅₋₁₀(N)₁₃₋₂₅AGUCUUGGACUUGUCCAUAUGACAA-3' (SEQ ID No: 2).

2. The double-stranded RNA according to claim 1, wherein none of the bases of the (N)₁₃₋₂₅ portions are complementary bases.

3. The double-stranded RNA according to claim 1, wherein all of the bases of the (N)₁₃₋₂₅ portions are complementary bases.

4. The double-stranded RNA according to any one of claims 1 to 3, wherein the Tm value is 20°C.

5. The double-stranded RNA according to claim 1, wherein the (N)₁₃₋₂₅ is (N)₂₅.

6. The double-stranded RNA according to claim 5, wherein none of the bases of the (N)₂₅ portions are complementary bases.

7. The double-stranded RNA according to claim 5, wherein all of the bases of the (N)₂₅ portions are complementary bases.

8. The double-stranded RNA according to any one of claims 5 to 7, wherein the (N)₅₋₁₀ is (N)₇.

9. The double-stranded RNA according to claim 8, wherein the Tm value is 20°C.

10. A pharmaceutical for immunostimulation comprising:
the double-stranded RNA according to any one of claims 1 to 9.

11. The pharmaceutical according to claim 10, wherein the immunostimulation is activation of natural killer cells.

12. A pharmaceutical for suppressing growth of cancer cells or tumor cells, the pharmaceutical comprising:
the double-stranded RNA according to any one of claims 1 to 9.

13. Double-stranded RNA composed of, or a pharmacologically acceptable salt thereof:
RNA composed of a base sequence represented by a sequence A below; and
RNA composed of a base sequence represented by a sequence B,
the double-stranded RNA or the pharmacologically acceptable salt thereof having an immunostimulatory activity or an activity of suppressing growth of cancer cells or tumor cells,
each base of an (N)₁₃₋₂₅ portion in the base sequence represented by the sequence A and the base sequence represented by the sequence B being or not being complementary bases, and
all of the bases of (N)₅₋₁₀ portions in the base sequence represented by the sequence A and the base sequence represented by the sequence B being complementary bases, and a Tm value of the (N)₅₋₁₀ portion being 20°C or higher:
Sequence A: 5'-UUGUCAUAUGGACAAGUCCAAGACU(N)₁₃₋₂₅(N)₅₋₁₀-3' (SEQ ID No: 1), and
Sequence B: 5'-(N)₅₋₁₀(N)₁₃₋₂₅AGUCUUGGACUUGUCCAUAUGACAA-3' (SEQ ID No: 2).

14. An injector injecting a solution containing biomolecules into an injection target from an injector body without performing injection through a predetermined structure in a state where the predetermined structure is inserted into the injection target, the injector comprising:
an accommodation unit that accommodates a solution containing biomolecules; and
a nozzle portion having an ejection port, through which a pressurized solution containing the biomolecules flows and is ejected to the injection target, wherein
the solution containing the biomolecules is a solution containing the double-stranded RNA according to any one of claims 1 to 9, the pharmaceutical according to any one of claims 10 to 12, or a solution containing the double-stranded RNA or a pharmacologically acceptable salt thereof according to claim 13.
